# EUROPEAN PATENT APPLICATION

(11) **EP 4 071 072 A1**
(43) Date of publication of application: **12.10.2022**
(21) Application number: 20899321.2
(22) Date of filing: 08.12.2020
(51) Int. Cl.: B65D 5/66, A61J 1/00, B65D 5/18

(54) **PACKAGING BOX AND MEDICAL EQUIPMENT PACKAGED BY PACKAGING BOX**

(30) Priority: 10.12.2019 JP 2019223316
(71) Applicant: TERUMO Kabushiki Kaisha, Tokyo 151-0072 (JP)
(72) Inventor: TONO Yohei, Nakakoma-gun, Yamanashi 409-3853 (JP); HASUMI Kiyoaki, Nakakoma-gun, Yamanashi 409-3853 (JP); OTSU Shinnosuke, Nakakoma-gun, Yamanashi 409-3853 (JP)
(74) Representative: Casalonga
(86) International application number: PCT/JP2020/045652
(87) International publication number: WO 2021/117715

(57) **Abstract**

A packaging box includes a body and a lid. The body includes a bottom surface part, a front surface part rising from the bottom surface part, and protruding pieces positioned at one side portion of the front surface part and extending from an upper edge of the front surface part. The lid includes a top surface part, a lid front surface part, lid side surface parts opposed to each other, and two lid front side joints joined to inner surfaces of both sides of the lid front surface parts. The lid front side joints have cutout portions at the upper end portion, bendable portions below the cutout portion, and a non-joint portion formed between the bendable portions and the lid front surface part, into which the distal ends of the protruding pieces can enter. The lid closed state is maintained by engagement between the protruding pieces and the bendable portions.

## Description

### BACKGROUND

### Technical Field

The present invention relates to a packaging box including a lid and a body connected to each other and capable of opening and closing the lid, and a medical instrument packaged in the packaging box.

### Related Art

Conventionally, a rectangular parallelepiped packaging box in which an opening line is broken and opened, and a lid is openable and closable after the opening, that is, a so-called flip top type packaging box, has been used as a container for products such as confectionery and cigarettes because a stored object is easily taken out.

JP 6066702 B2 and JP 4882772 B2 have been proposed as the flip top type capable of closing a lid to bring the lid into a lid closed state after opening.

Paragraph 0035 of JP 6066702 B2 discloses the following.

(0035) A finger F is inserted into a cutout 45 to move one piece of wrapped butter W in the direction Z1 to move outward through the opening 10a.

After taking out the one piece of wrapped butter W from the paper box container 1, the lid 50 pivots toward the body 10 side at the hinge fold 16 (the lid 50 is closed).

Since the first inner surface panel 12 is provided with a rounded corner 23 and the second inner surface panel 14 is provided with a rounded corner 43, the lid 50 is prevented from being locked to the inner surface panels 12 and 14 when the lid 50 pivots. Similarly, since the outer surface of the tongue 24 is the inclined surface as described above, the flap panel 47 abutting on the outer surface of the tongue 24 is guided along the outer surface of the tongue 24 as the top panel 51 approaches the first inner surface panel 12, and the flap panel 47 is prevented from being locked to the outer surface of the tongue 24.

When the flap panel 47 of the lid 50 gets over the tongue 24 of the body 10, the other end 24b of the tongue 24 is deformed so as to be separated from the first inner surface panel 12 by the elastic force of the tongue 24, and the other end 24b of the tongue 24 abuts on the inner surface of the first outer surface panel 52. As a result, a notch 47a of the flap panel 47 is reliably locked to the other end 24b of the tongue 24.

Paragraph 0041 of JP 6066702 B2 discloses the following.

(0041) The paper box container 1 includes the tongue 24 and the flap panel 47. In a state where the lid 50 is closed, the notch 47a of the flap panel 47 is locked to the other end 24b of the tongue 24, and the lid 50 is prevented from being inadvertently opened.

An inner surface side slit 21 is formed in the first inner surface panel 12, and an insertion piece 27 is provided in the first outer surface panel 52. When the insertion piece 27 is inserted through the inner surface side slit 21 in a state where the lid 50 is closed, the first outer surface panel 5 disposed at a base end of the insertion piece 27 is locked to an edge of the inner surface side slit 21 when the lid 50 is opened. Therefore, the lid 50 can be prevented from being opened.

Paragraph 0019 of JP 4882772 B2 discloses the following.

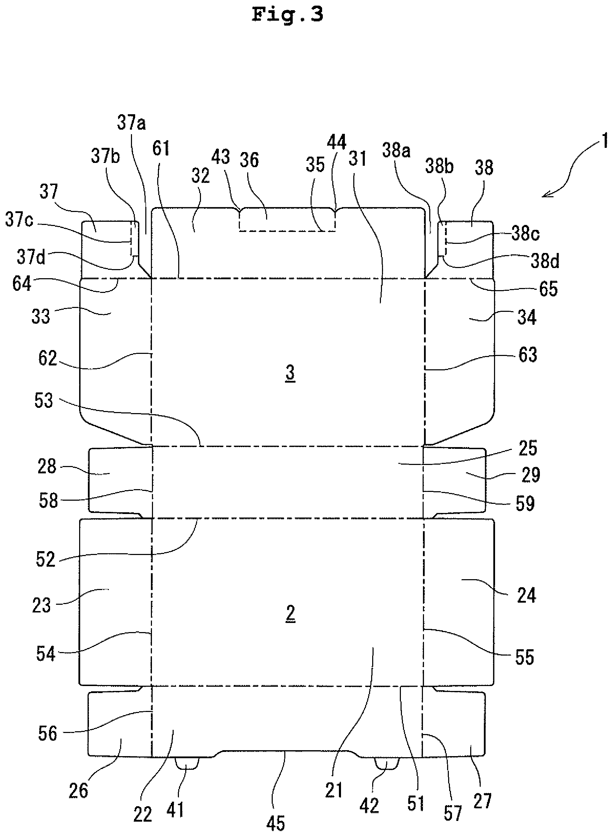

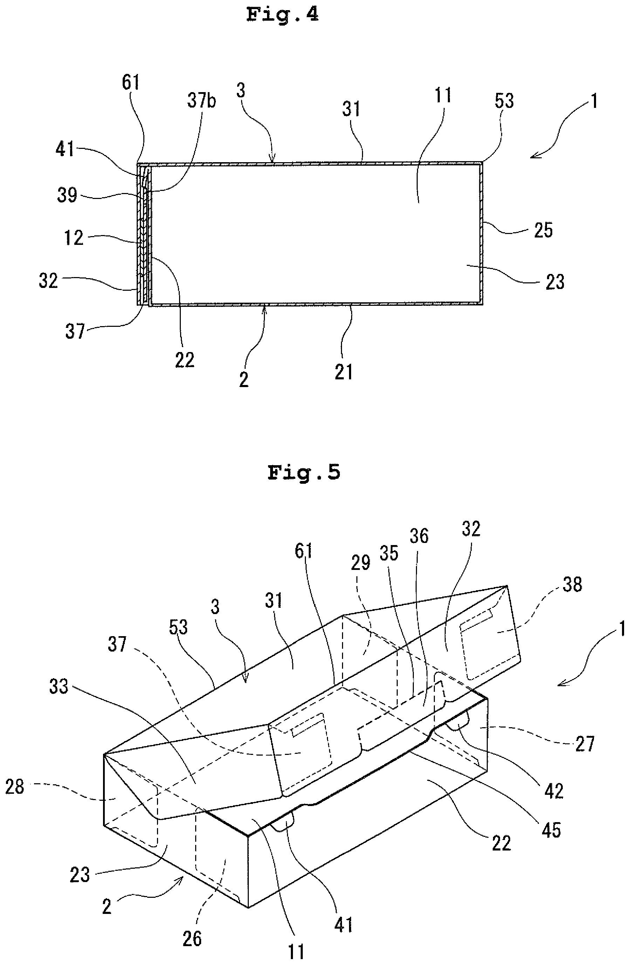

(0019) FIG. 3 is an explanatory perspective view illustrating an opening completion state of the first embodiment, and when a re-closable paper box of the present invention is used, the operation of pulling up the lid, specifically, the operation of peeling off the lid front panel 1 from the front panel 5 breaks a front surface breaking line X between a lid overlapping sticking panel 13 stuck to the inner surface of the lid front panel 1 and a fold-back locking piece 12, and breaks side surface inclined break lines Y and Z punched on upper ends of left and right overlapping side panels 8 and 9, causing a lid formed in a cover shape that is constituted of left and right overlapping side panel sticking parts 8' and 9' to which the lid front panel 1 and the left and right lid side panels 10 and 11 are stuck, a top panel 2, and a lid rear panel 3' to open in a double-sided opening shape with a vertical opening horizontal folding line L12 provided at an upper end of a rear panel 3 as a hinge portion. After the use of the stored or filled contents, the lid formed in the cover shape can be reclosed by being returned to an original position, and as illustrated in an explanatory partial cross-sectional view illustrating a reclose completion state of the embodiment of FIG. 4, the front surface breaking line X that is stuck to the inner surface of the lid front panel 1 is broken by the opening operation, and the upper end breaking portion end surfaces of the portions of the fold-back locking pieces 12 at both ends of the lid overlapping sticking panel 13 that are broken, are locked to the lower end breaking portion end surfaces of the fold-back locking pieces 12 and 12 folded back from both ends of the upper end of the front panel 5 constituting a frame part, and stable reclosing is maintained.

### PRIOR ART DOCUMENTS

### PATENT DOCUMENTS

Patent Document 1: Japanese Patent No. 6066702
Patent Document 2: Japanese Patent No. 4882772

### SUMMARY

### PROBLEMS TO BE SOLVED BY THE INVENTION

In the boxes of JP 6066702 B2 and JP 4882772 B2, the engagement between the lid and the body at the time of reclosing is performed only by the abutment between the end surfaces of the tongue (fold-back locking piece) and the notch (upper end breaking portion end surface).

Therefore, when the side portion of the first inner surface (the upper portion of the front panel) is slightly pushed, the end surface engagement described above is easily released. Therefore, in the boxes of JP 6066702 B2 and JP 4882772 B2, it is difficult to maintain a stable lid closed state.

Therefore, the present invention provides a packaging box from which a stored object is easily taken out from a body after opening a lid, and by closing the lid, the lid can be reliably brought into a lid closed state and the lid closed state can be reliably maintained, and a packaged medical instrument using the packaging box.

### MEANS FOR SOLVING THE PROBLEMS

The above object is achieved by the following.

A packaging box includes: a body having a storage whose upper side is opened; and a lid connected to the body and capable of opening and closing the opening, the packaging box capable of storing a plurality of stored objects, in which
the body includes a bottom surface part, a front surface part rising from the bottom surface part, two side surface parts opposed to each other rising from the bottom surface part, and a back surface part rising from the bottom surface part and opposed to the front surface part,
the lid includes a top surface part, a lid front surface part extending downward from the top surface part, two lid side surface parts opposed to each other extending downward from the top surface part, and two lid front side joints extending from tips of the two lid side surface parts, and the two lid front side joints are connected to the inner surfaces of both side portions of the lid front surface part,
when the lid is closed, the lid front surface part is positioned on a surface side of the front surface part of the body,
the body further includes a protruding piece positioned on one side portion of the front surface part and extending from an upper edge of the front surface part,
the lid front side joint includes a cutout portion provided at an upper end portion and extending from a base end side of the lid front side joint toward a distal end side by a predetermined length, and a bendable portion provided below the cutout portion, and a portion between the bendable portion and the lid front surface part is a non-joint portion into which a distal end of the protruding piece of the body is able to enter, and
in the packaging box, when the lid is closed or when operating to open the lid, the protruding piece of the body enters the non-joint portion, and the protruding piece and the bendable portion abut on each other to bring the lid into a lid locked state, and when operating to open the lid after entering of the protruding piece, the bendable portion of the lid front side joint is bent by abutment with the protruding piece, pushes up the protruding piece at a bendable portion, and is able to release a contact state between the lid and the protruding piece, and further, when operating to close the lid, the lid front side joint comes into contact with the protruding piece, pushes down the protruding piece, and the protruding piece enters the non-joint portion of the lid, and the protruding piece and the bendable portion are engaged with each other, and a lid closed state is maintained.

The above object is achieved by the following.

A packaging box includes: a body having a storage whose upper side is opened; and a lid connected to the body and capable of opening and closing the opening, the packaging box capable of storing a plurality of stored objects, in which
the body includes a bottom surface part, a front surface part rising from the bottom surface part, two side surface parts opposed to each other rising from the bottom surface part, and a back surface part rising from the bottom surface part and opposed to the front surface part,
the lid includes a top surface part, a lid front surface part extending downward from the top surface part, two lid side surface parts opposed to each other extending downward from the top surface part, and two lid front side joints extending from both ends of the lid front surface parts, and the two lid front side joints are joined to inner surfaces of the distal ends of the two lid side surface part,
when the lid is closed, the lid front surface part is positioned on a surface side of the front surface part of the body,
the body further includes a protruding piece positioned on a distal end of one of the side surface parts and extending from an upper edge of the side surface part,
the lid front side joint includes a cutout portion provided at an upper end portion and extending from a base end side toward a distal end side of the lid front side joint by a predetermined length, and a bendable portion provided below the cutout portion, and a portion between the bendable portion and the lid side surface part is a non-joint portion into which a distal end of the protruding piece of the body is able to enter, and
in the packaging box, when the lid is closed or when operating to open the lid, the protruding piece of the body enters the non-joint portion, and the protruding piece and the bendable portion abut on each other to bring the lid into a lid locked state, and when operating to open the lid after entering of the protruding piece, the bendable portion of the lid front side joint is bent by abutment with the protruding piece, pushes up the protruding piece at a bendable portion, and is able to release a contact state between the lid and the protruding piece, and further, when operating to close the lid, the lid front side joint comes into contact with the protruding piece, pushes down the protruding piece, and the protruding piece enters the non-joint portion of the lid, and the protruding piece and the bendable portion are engaged with each other, and a lid closed state is maintained.

The above object is achieved by the following.

A packaged medical instrument includes: the packaging box; and a plurality of medical instruments housed in the packaging box, in which the opening of the body of the packaging box is blocked by the lid, and the lid is in a state of being bonded to a surface portion of the front surface part of the body overlapping a pressing portion for opening on a rear surface of the pressing portion for opening.

### BRIEF DESCRIPTION OF DRAWINGS

FIG. 1 is a perspective view of a packaging box according to an embodiment of the present invention as viewed from an upper surface side;
FIG. 2 is a front view of the packaging box illustrated in FIG. 1;
FIG. 3 is a developed view of a packaging box according to an embodiment of the present invention;
FIG. 4 is a cross-sectional view taken along line A-A of FIG. 2;
FIG. 5 is a perspective view as viewed from an upper surface side in a state where a lid of the packaging box illustrated in FIG. 1 is opened;
FIG. 6 is a plan view of a state in which a lid of the packaging box illustrated in FIG. 2 stands upright;
FIG. 7 is a front view of a state where the lid of the packaging box illustrated in FIG. 2 is completely opened;
FIG. 8 is a right side view of the packaging box illustrated in FIG. 7;
FIG. 9 is an explanatory view for explaining an operation of the packaging box of the present invention;
FIG. 10 is an explanatory view for explaining an operation of the packaging box of the present invention;
FIG. 11 is an explanatory view for explaining an operation of the packaging box of the present invention;
FIG. 12 is an explanatory view for explaining an operation of the packaging box of the present invention;
FIG. 13 is a front view of a packaged medical instrument of an embodiment of the present invention;
FIG. 14 is a plan view of the packaged medical instrument of the present invention illustrated in FIG. 13;
FIG. 15 is a perspective view of the packaged medical instrument of the present invention as viewed from the upper surface side in a state where the packaged medical instrument is opened and the lid is slightly opened;
FIG. 16 is a plan view of a state where the packaged medical instrument illustrated in FIG. 13 is opened and the lid of the packaging box stands upright;
FIG. 17 is a developed view of a packaging box according to another embodiment of the present invention; and
FIG. 18 is a perspective view of the packaging box according to the other embodiment of the present invention as viewed from an upper surface side.

### DESCRIPTION OF EMBODIMENTS

A description of a packaging box of the present invention will hereinafter be given, using embodiments illustrated in the drawings.

A packaging box 1 of the present invention is a packaging box that includes a body 2 having a storage 11 whose upper side is opened, and a lid 3 connected to the body 2 and capable of opening and closing the opening, and that is capable of storing a plurality of stored objects.

The body 2 includes a bottom surface part 21, a front surface part 22 rising from the bottom surface part 21, two side surface parts 23 and 24 opposed to each other rising from the bottom surface part 21, and a back surface part 25 rising from the bottom surface part 21 and opposed to the front surface part 22. The lid 3 includes a top surface part 31, a lid front surface part 32 extending downward from the top surface part 31, two lid side surface parts 33 and 34 opposed to each other extending downward from the top surface part, and two lid front side joints 37 and 38 extending from tips of the two lid side surface parts 33 and 34, and the two lid front side joints 37 and 38 are joined to inner surfaces of both side portions of the lid front surface part 32.

When the lid 3 is closed, the lid front surface part 32 is positioned on the front surface side of the front surface part 22 of the body 2. The front surface part 22 of the body 2 includes protruding pieces 41 and 42 positioned on one side portion of the front surface part 22 and extending from the upper edge of the front surface part 22, the lid front side joints 37 and 38 include cutout portions 37a and 38a provided at the upper end portion and extending from the base end side of the lid front side joints 37 and 38 toward the distal end side by a predetermined length, and bendable portions 37b and 38b provided below the cutout portions 37a and 38a, and a portion between the bendable portions 37b and 38b and the lid front surface part 32 is a non-joint portion 39 into which the distal ends of the protruding pieces 41 and 42 of the body 2 are able to enter.

In the packaging box 1, the protruding pieces 41 and 42 of the body 2 enter the non-joint portion 39 when the lid 3 is closed or when operating to open the lid 3, and the protruding pieces 41 and 42 and the bendable portions 37b and 38b abut on each other to be in the lid locked state, and the bendable portions 37b and 38b of the lid front side joints 37 and 38 are bent by abutting on the protruding pieces 41 and 42 when operating to open the lid after the entering of the protruding pieces 41 and 42 (during a lid opening operation), and push up the protruding pieces 41 and 42 at the bendable portions to release the contact state (engagement state between the protruding pieces 41 and 42 and the bendable portions 37b and 38b) between the lid 3 and the protruding pieces 41 and 42, and further, the lid front side joints 37 and 38 are in contact with the protruding pieces 41 and 42 when operating to close the lid 3 (during a lid closing operation), and push down the protruding pieces 41 and 42, and the protruding pieces 41 and 42 enter the non-joint portion 39 of the lid 3, and the protruding pieces 41 and 42 and the bendable portions 37b and 38b are engaged with each other to maintain the lid closed state.

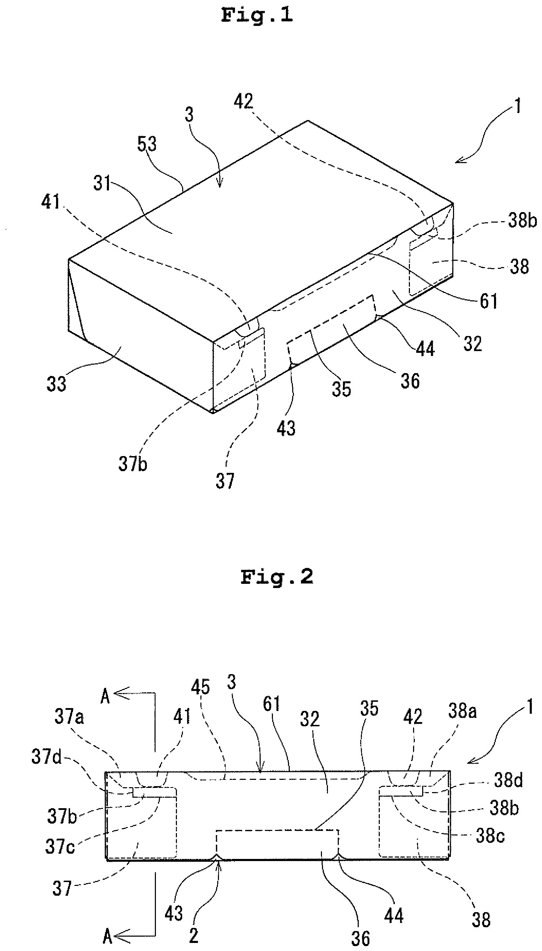

As illustrated in FIGS. 1 to 8, the packaging box 1 of the present invention includes a rectangular parallelepiped body 2 having an open upper side, and a lid 3 connected to the body 2 and capable of closing the opening.

The body 2 includes, as illustrated in FIGS. 1 to 8, the bottom surface part 21, the front surface part 22 rising from the bottom surface part 21, the back surface part 25 rising from the bottom surface part 21 and opposed to the front surface part 22, and the two side surface parts 23 and 24 opposed to each other rising from the bottom surface part 21. The two side surface parts 23 and 24 are joined to the front surface part 22 and the back surface part 25. In this embodiment, the body 2 has a rectangular parallelepiped shape including a predetermined capacity of the storage 11 therein.

The front surface part 22 of the body 2 includes protruding pieces 41 and 42 positioned on one side portion (in this embodiment, one end portion and the other end portion) of the front surface part 22 and extending from an upper edge of the front surface part 22. It is preferable that two protruding pieces are provided, but only one of the protruding pieces may be provided. The protruding pieces 41 and 42 are separated from one end and the other end of the upper edge of the front surface part 22, and are provided at positions slightly on the center side. The protruding pieces 41 and 42 are provided at positions corresponding to the bendable portions 37b and 38b of the lid front side joints 37 and 38. The width of each of the protruding pieces 41 and 42 decreases in the distal end direction. In addition, the protruding pieces 41 and 42 have a shape in which both side portions of the distal end are chamfered. In other words, the protruding pieces 41 and 42 have a substantially trapezoidal shape with both corners of the distal ends chamfered. The protruding pieces 41 and 42 each have a linear portion at the center of the tip.

Furthermore, in this embodiment, a cutout portion 45 is provided in a central portion of the front surface part 22 of the body in order to facilitate the taking out of an internal stored object, particularly a stored object positioned close to the front surface part 22. In the cutout portion 45, a side surface of the internal stored object is exposed, and thus the internal stored object can be easily held and taken out.

In addition, in this embodiment, as illustrated in FIGS. 4 and 5, the body 2 extends from the front surface part 22 or the side surface parts 23 and 24, and includes two front side joints 26 and 27 to join the front surface part 22 and the side surface parts 23 and 24. In particular, in this embodiment, the body 2 includes the two front side joints 26 and 27 extending from both side portions of the front surface part 22 to join the side surface parts 23 and 24, and outer surfaces of the two front side joints 26 and 27 are joined to inner surfaces of the side surface parts 23 and 24.

In addition, as illustrated in FIGS. 4 and 5, the body 2 includes two back side joints 28 and 29 extending from the back surface part 25 or the side surface parts 23 and 24, to join the back surface part 25 and the side surface parts 23 and 24. In particular, in this embodiment, the body 2 includes the two back side joints 28 and 29 extending from both sides of the back surface part 25 to join the side surface parts 23 and 24, and outer surfaces of the two back side joints 28 and 29 are joined to inner surfaces of the side surface parts 23 and 24.

In addition, in this embodiment, the two front side joints 26 and 27 are provided, at the lower ends, with cutout portions extending in the base end direction (front surface part direction) from the tips. Similarly, the two back side joints 28 and 29 are provided, at the lower ends, with cutout portions extending in the base end direction (back surface part direction) from the tips. In addition, in this embodiment, the front side joints 26 and 27 and the back side joints 28 and 29 have a substantially trapezoidal shape whose width decreases toward the tip.

The body 2 is bent (valley folded) along a fold line 51 in FIG. 3, and the front surface part 22 rises from the bottom surface part 21. The body 2 is bent (valley folded) along fold lines 54 and 55, and the side surface parts 23 and 24 rise from the bottom surface part 21. The body 2 is bent (valley folded) along a fold line 52, and the back surface part 25 rises from the bottom surface part 21. The two front side joints 26 and 27 are bent (valley folded) along fold lines 56 and 57 and disposed inside the side surface parts 23 and 24. Similarly, the two back side joints 28 and 29 are bent (valley folded) along fold lines 58 and 59, and are disposed inside the side surface parts 23 and 24. Then, the two front side joints 26 and 27 are jointed to the inside of the side surface parts 23 and 24, and the two back side joints 28 and 29 are jointed to the inside of the side surface parts 23 and 24, and thus the body 2 is formed.

As illustrated in FIGS. 1 to 8, the lid 3 includes the top surface part 31, the lid front surface part 32 extending downward from the top surface part 31, and the two lid side surface parts 33 and 34 opposed to each other and extending downward from the top surface part 31. The two lid side surface parts 33 and 34 further include the two lid front side joints 37 and 38 extending from the tips of the two lid side surface parts 33 and 34 and joined to the inner surfaces of both side portions of the lid front surface part 32, and the two lid front side joints 37 and 38 are joined to the inner surface of the lid front surface part 32. Thus, the lid 3 is formed.

In this embodiment, the rear ends of the two lid side surface parts 33 and 34 are inclined edges inclined toward the distal end side, and the corners of the rear ends of the two lid side surface parts 33 and 34 are chamfered and rounded.

The lid 3 is connected to the body 2 via a hinge line 53. Specifically, the body 2 and the lid 3 are provided with the hinge line 53 at a boundary portion between the back surface part 25 of the body 2 and the top surface part 31 of the lid 3, and are pivotally connected to each other via the hinge line 53. In other words, the body 2 and the lid 3 are connected to each other by connecting the upper end of the back surface part 25 of the body 2 and the rear end of the top surface part 31 of the lid 3. The lid 3 pivots about the connecting part (hinge line) 53, and can open and close the body 2.

The top surface part 31 of the lid 3 is slightly larger than the bottom surface part 21, and is capable of closing the upper opening of the body 2. When the lid 3 is put on the body 2, the lid front surface part 32 of the lid 3 is positioned on the front side of the front surface part 22 of the body 2, and similarly, the lid side surface parts 33 and 34 of the lid 3 are positioned on the front sides of the side surface parts 23 and 24 of the body 2. Therefore, the lid 3 covers the body 2 from the outside.

The lid front surface part 32 includes a breaking slit 35 whose distal and terminal ends are positioned at the lower end of the lid front surface part 32, and a pressing portion for opening 36 defined by the breaking slit 35 and the lower end of the lid front surface part 32. In a state of covering the body 2, the lid 3 is bonded at the rear surface of the pressing portion for opening 36 and a surface portion of the front surface part 22 of the body 2 overlapping the pressing portion for opening 36.

Furthermore, in this embodiment, the lid front surface part 32 includes small cutout portions 43 and 44 positioned on both sides of the lower end of the pressing portion for opening 36. In other words, the small cutout portions 43 and 44 positioned at the distal and the terminal ends of the breaking slit 35 are provided. By providing the small cutout portions 43 and 44, the start of breaking is improved.

The lid 3 is bent (valley folded) along a fold line 61 in FIG. 3, and the lid front surface part 32 rises from the top surface part 31. The lid 3 is bent (valley folded) along fold lines 62 and 63, and the lid side surface parts 33 and 34 rise from the top surface part 31. The two lid front side joints 37 and 38 are bent (valley folded) along fold lines 64 and 65, and are disposed inside the lid side surface parts 33 and 34. Then, the lid 3 is formed by joining the two lid front side joints 37 and 38 to the inside of the lid front surface part 32.

As illustrated in FIGS. 1 to 8, the lid front side joints 37 and 38 are provided at the upper end portions, and include the cutout portions 37a and 38a extending from the base end side of the lid front side joints 37 and 38 toward the distal end side by a predetermined length. In this embodiment, the cutout portion 37a is formed by a linear part substantially parallel to the distal end edge (fold line 61) of the top surface part 31 and an inclined line connecting the base end of the linear part and the base end upper portion (one end of the fold line 61) of the lid front side joint 37. Similarly, in this embodiment, the cutout portion 38a is formed by a linear part substantially parallel to the distal end edge (fold line 61) of the top surface part 31 and an inclined line connecting the base end of the linear part and the base end upper portion (the other end of the fold line 61) of the lid front side joint 38. The widths (distance between the linear part of the cutout portions 37a and 38a and the fold line 61) of the cutout portions 37a and 38a of the lid front side joints 37 and 38 are preferably about the same as the protruding lengths of the protruding pieces 41 and 42 of the body 2 described above, and specifically, are preferably the same as, slightly shorter, or slightly longer than these.

As illustrated in FIGS. 1 to 8, the lid front side joints 37 and 38 include the bendable portions 37b and 38b provided below the cutout portions 37a and 38a. Between the bendable portions 37b and 38b and the lid front surface part 32, the non-joint portion 39 into which the distal ends of the protruding pieces 41 and 42 of the body 2 are able to enter is formed. Specifically, the bendable portions 37b and 38b are formed by linear easily deformable portions (lateral linear easily deformable portions) 37c and 38c formed substantially parallel to the upper edges (linear parts) of the cutout portions 37a and 38a, and linear easily deformable portions (longitudinal linear easily deformable portions) 37d and 38d extending from the base end side portions of the linear easily deformable portions 37c and 38c to the upper edges. In this embodiment, the linear easily deformable portions 37c and 38c are formed by perforations. Furthermore, the linear easily deformable portions (longitudinal linear easily deformable portions) 37d and 38d are preferably formed by perforation, but may be slits.

In this embodiment, the bendable portions 37b and 38b have a rectangular shape extending by a predetermined length toward the base end side from the tips of the lid front side joints 37 and 38 and substantially parallel to the distal end edge (fold line 61) of the top surface part 31. Furthermore, the bendable portions 37b and 38b include the linear easily deformable portions (perforations) 37c and 38c extending from the tips of the cutout portions 37a and 38a, extending slightly before the terminal ends of the linear parts, and forming the lower ends of the bendable portions 37b and 38b. Furthermore, the bendable portions 37b and 38b include the linear easily deformable portions (longitudinal linear easily deformable portions) 37d and 38d extending toward the linear part from the base end portions of the linear easily deformable portions (perforations) 37c and 38c and reaching the linear part. The bendable portions 37b and 38b are easily deformed because the inner edge portions positioned in the lid front side joints 37 and 38 are formed of the linear easily deformable portions (perforations) 37c and 38c and the linear easily deformable portions (longitudinal linear easily deformable portions) 37d and 38d.

Furthermore, as illustrated in FIG. 4, the lid front side joints 37 and 38 are joined (fixed) to the lid front surface part 32 by a joint 12 at a portion below the bendable portions 37b and 38b [linear easily deformable portions (lateral linear easily deformable portions) 37c and 38c, linear easily deformable portions (longitudinal linear easily deformable portions) 37d and 38d], and a portion between the bendable portions 37b and 38b and the lid front surface part 32 is the non-joint portion 39. Therefore, the joint 12 does not inhibit deformation of the bendable portions 37b and 38b. In addition, since the joint 12 has a certain thickness, there is a slight gap between the bendable portions 37b and 38b and the non-joint portion 39 of the lid front surface part 32, and the protruding pieces 41 and 42 of the body 2 are able to enter.

As illustrated in FIG. 4, in the packaging box of this embodiment, when the lid 3 is closed or when operating to open the lid 3, the protruding pieces 41 and 42 of the body 2 enter the non-joint portion 39, and the protruding pieces 41 and 42 and the bendable portions 37b and 38b come into contact with each other, and the lid locked state is obtained. After the protruding pieces 41 and 42 enter, at the time of the operation of opening the lid (during the lid opening operation), the bendable portions 37b and 38b of the lid front side joints 37 and 38 are bent by abutting on the protruding pieces 41 and 42, and the protruding pieces 41 and 42 are pushed up at the bendable portions, and a contact state (engagement state between the protruding pieces 41 and 42 and the bendable portions 37b and 38b) between the lid 3 and the protruding pieces 41 and 42 can be released. Furthermore, at the time of the operation of closing the lid 3 (during the lid closing operation), the lid front side joints 37 and 38 come into contact with the protruding pieces 41 and 42 to push down the protruding pieces 41 and 42, and the protruding pieces 41 and 42 enter the non-joint portion 39 of the lid 3, and the protruding pieces 41 and 42 and the bendable portions 37b and 38b are engaged with each other to maintain the lid closed state.

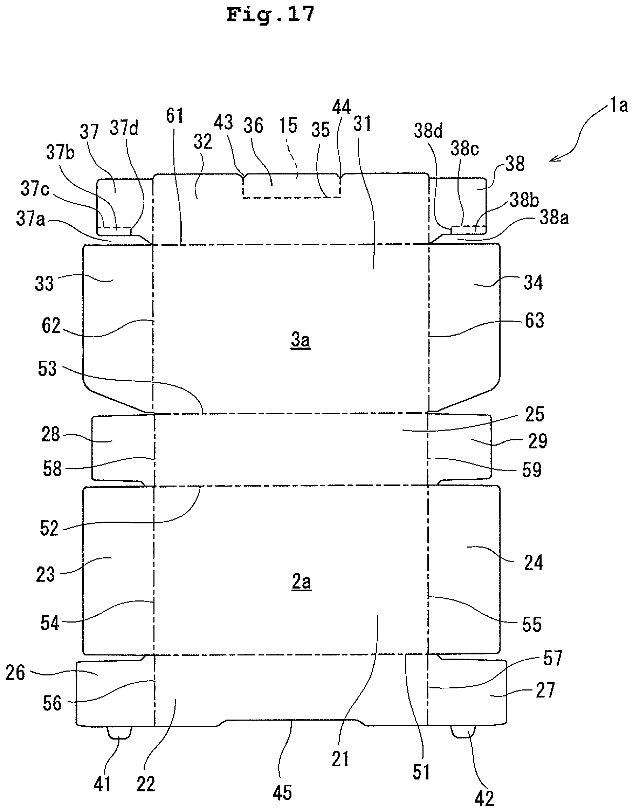

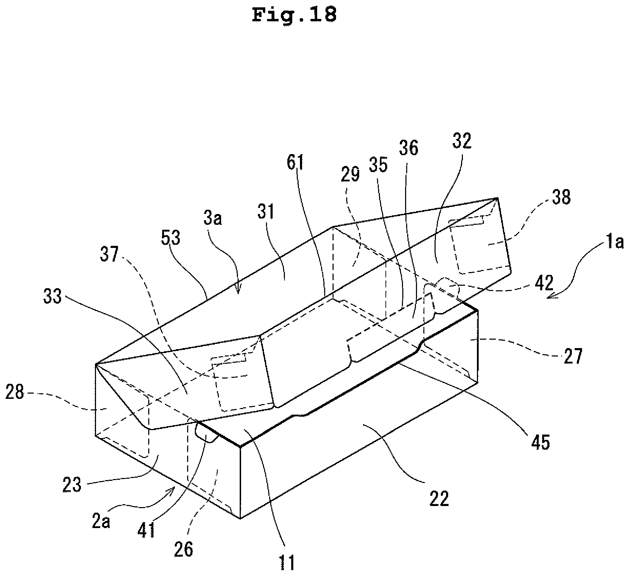

Next, a packaging box according to another embodiment of the present invention illustrated in FIGS. 17 and 18 will be described. FIG. 17 is a developed view of another embodiment of the present invention. FIG. 18 is a perspective view of the packaging box of the other embodiment of the present invention as viewed from an upper surface side.

A packaging box 1a of this embodiment has the same basic configuration as the packaging box described above. The main differences are the arrangement positions of the protruding pieces 41 and 42 and the arrangement positions of the lid front side joints 37 and 38.

The packaging box 1a of this embodiment is a packaging box that includes a body 2a having a storage 11 whose upper side is opened, and a lid 3a connected to the body 2a and capable of opening and closing the opening, and that is capable of storing a plurality of stored objects.

The body 2a includes a bottom surface part 21, a front surface part 22 rising from the bottom surface part 21, two side surface parts 23 and 24 opposed to each other rising from the bottom surface part 21, and a back surface part 25 rising from the bottom surface part 21 and opposed to the front surface part 22. The lid 3a includes a top surface part 31, a lid front surface part 32 extending downward from the top surface part 31, and two lid side surface parts 33 and 34 opposed to each other extending downward from the top surface part.

The packaging box 1a of this embodiment includes two lid front side joints 37 and 38 extending from both ends of the lid front surface part 32, and the two lid front side joints 37 and 38 are joined to the inner surfaces of the distal ends of the two lid side surface parts 33 and 34.

When the lid 3a is closed, the lid front surface part 32 is positioned on the front surface side of the front surface part 22 of the body 2a. In an assembled state, the body 2a includes protruding pieces 41 and 42 that are positioned at a distal end (in this embodiment, the distal ends of both side surface parts 23 and 24) of one side surface part and extend from upper edges of the side surface parts 23 and 24.

In the packaging box 1a of this embodiment, the protruding pieces 41 and 42 are formed in the front side joints 26 and 27, and in a state where the two front side joints 26 and 27 are joined to the inside of the side surface parts 23 and 24, the protruding pieces 41 and 42 extend from the upper edges of the side surface parts 23 and 24.

The body 2 is bent (valley folded) along a fold line 51 in FIG. 3, and the front surface part 22 rises from the bottom surface part 21. The body 2 is bent (valley folded) along fold lines 54 and 55, and the side surface parts 23 and 24 rise from the bottom surface part 21. The body 2 is bent (valley folded) along a fold line 52, and the back surface part 25 rises from the bottom surface part 21. The two front side joints 26 and 27 are bent (valley folded) along fold lines 56 and 57 and disposed inside the side surface parts 23 and 24. Similarly, the two back side joints 28 and 29 are bent (valley folded) along fold lines 58 and 59, and are disposed inside the side surface parts 23 and 24. Then, the two front side joints 26 and 27 are jointed to the inside of the side surface parts 23 and 24, and the two back side joints 28 and 29 are jointed to the inside of the side surface parts 23 and 24, and thus the body 2 is formed.

The lid front side joints 37 and 38 include cutout portions 37a and 38a provided at the upper end portion and extending from the base end side of the lid front side joints 37 and 38 toward the distal end side by a predetermined length, and bendable portions 37b and 38b provided below the cutout portions 37a and 38a, and a portion between the bendable portions 37b and 38b and the lid side surface parts 33 and 34 is a non-joint portion 39 into which the distal ends of the protruding pieces 41 and 42 of the body 2a are able to enter.

The packaging box 1a of this embodiment and the packaging box 1 described above have the same basic configuration, but the arrangement portions of the protruding pieces 41 and 42 and the arrangement positions of the lid front side joints 37 and 38 are different.

In the packaging box 1a of this embodiment, as illustrated in FIGS. 17 and 18, the lid front side joints 37 and 38 extend from both ends of the lid front surface part 32, and the lid front side joints 37 and 38 are joined to the inner surfaces of the distal ends of the two lid side surface parts 33 and 34. Therefore, the lid front side joints 37 and 38 are positioned on the side surface of the lid 3a. Also in this embodiment, it is preferable that the lid front side joints 37 and 38 have the same configuration as that described in the packaging box 1 described above. Note that, in this embodiment, a portion between the bendable portions 37b and 38b and the lid side surface parts 33 and 34 is the non-joint portion 39 into which the distal ends of the protruding pieces 41 and 42 of the body 2a are able to enter.

In the packaging box 1a of this embodiment, the body 2a includes the protruding pieces 41 and 42 provided at the distal ends of both the side surface parts 23 and 24, specifically, at positions corresponding to the bendable portions 37b and 38b of the lid front side joints 37 and 38 so as to correspond to the arrangement positions of the lid front side joints 37 and 38. Therefore, the protruding pieces 41 and 42 are positioned on the side surface of the body 2a. Also in this embodiment, the protruding pieces 41 and 42 preferably have the same configuration as that described in the packaging box 1 described above.

Also in the packaging box 1a of this embodiment, the protruding pieces 41 and 42 of the body 2a enter the non-joint portion 39 when the lid 3a is closed or when operating to open the lid 3a, and the protruding pieces 41 and 42 and the bendable portions 37b and 38b abut on each other to be in the lid locked state, and the bendable portions 37b and 38b of the lid front side joints 37 and 38 are bent by abutting on the protruding pieces 41 and 42 when operating to open the lid after the entering of the protruding pieces 41 and 42 (during the lid opening operation), and push up the protruding pieces 41 and 42 at the bendable portions to release the contact state (engagement state between the protruding pieces 41 and 42 and the bendable portions 37b and 38b) between the lid 3 and the protruding pieces 41 and 42, and further, the lid front side joints 37 and 38 are in contact with the protruding pieces 41 and 42 when operating to close the lid 3a (during the lid closing operation), and push down the protruding pieces 41 and 42, and the protruding pieces 41 and 42 enter the non-joint portion 39 of the lid 3a, and the protruding pieces 41 and 42 and the bendable portions 37b and 38b are engaged with each other to maintain the lid closed state.

An operation of the packaging box 1 of the present invention will be described with reference to FIGS. 4 to 12.

As illustrated in FIG. 4, in the packaging box 1 of this embodiment, in a state where the lid 3 is closed and the opening of the body 2 is closed, the protruding pieces 41 and 42 of the body 2 approach or abut on the upper portions of the upper edges of the bendable portions 37b and 38b. In a state where the lid 3 is closed and the opening of the body 2 is closed, the protruding pieces 41 and 42 of the body 2 may enter the non-joint portion 39 formed between the bendable portions 37b and 38b and the lid front surface part 32.

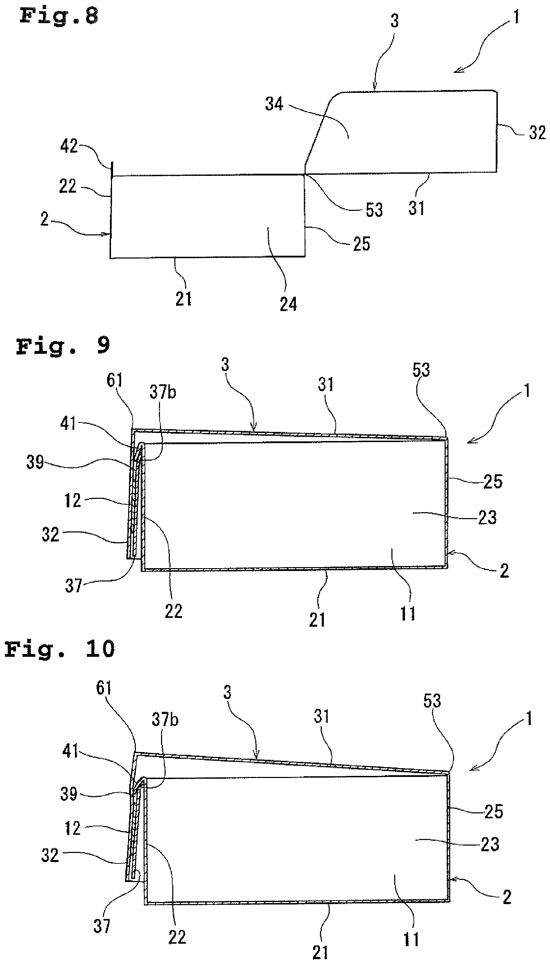

As illustrated in FIG. 9, in the packaging box 1 of this embodiment, when the lid 3 slightly pivots in the opening direction (in other words, immediately after the start of the operation of opening the lid 3), the bendable portions 37b and 38b of the lid 3 move upward, and thus the protruding pieces 41 and 42 of the body 2 enter the non-joint portion 39. The lower surfaces of the protruding pieces 41 and 42 abut on the upper ends of the bendable portions 37b and 38b. As a result, the lid 3 is locked to the body 2 (lid locked state).

As illustrated in FIG. 10, when the lid 3 further pivots in the opening direction (in other words, while the operation of opening the lid 3 is continued), the bendable portions 37b and 38b of the lid 3 further move upward, the bendable portions 37b and 38b are bent and push up the protruding pieces 41 and 42, and the distal end sides of the protruding pieces 41 and 42 also move upward and are separated from the front surface part 22.

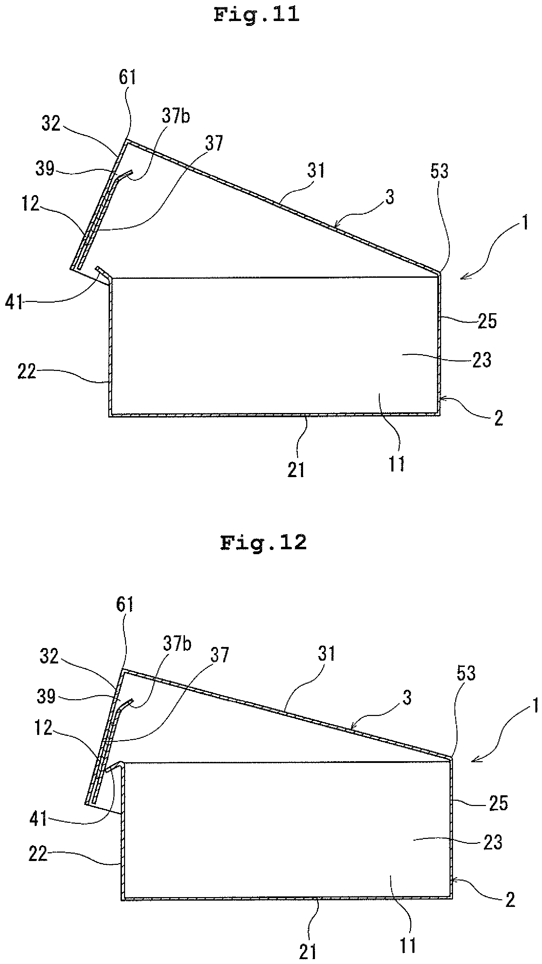

As illustrated in FIGS. 11 and 10, when the lid 3 further pivots in the opening direction (in other words, while the operation of opening the lid 3 is continued), the bendable portions 37b and 38b of the lid 3 further move upward, push up the protruding pieces 41 and 42, and are separated from the protruding pieces 41 and 42. As a result, the engagement state between the bendable portions 37b and 38b of the lid 3 and the protruding pieces 41 and 42 of the body 2, that is, the lid locked state is released.

Then, as illustrated in FIG. 12, for example, when the lid 3 pivots in the closing direction from the state in FIG. 11 (in other words, while the lid is pressed downward and the lid closing operation is performed), the lid front side joints 37 and 38 come into contact with the protruding pieces 41 and 42 and push down the protruding pieces 41 and 42. By further pivoting the lid 3 in the closing direction (continued downward pressing and continued lid closing operation), the bendable portions 37b and 38b of the lid 3 further move downward and pass through the protruding pieces 41 and 42.

Then, by ending the downward pressing (pushing up) of the lid 3, the state illustrated in FIG. 9 is obtained, and the protruding pieces 41 and 42 of the body 2 enter the non-joint portion 39 of the lid 3. The upper ends of the bendable portions 37b and 38b abut on the lower surfaces of the protruding pieces 41 and 42, and the lid 3 is locked to the body 2 (lid locked state). There is a possibility that the state illustrated in FIG. 4 is obtained by ending the downward pressing (pushing up) of the lid 3, but when the lid 3 moves even slightly upward, the lid locked state as illustrated in FIG. 9 is obtained.

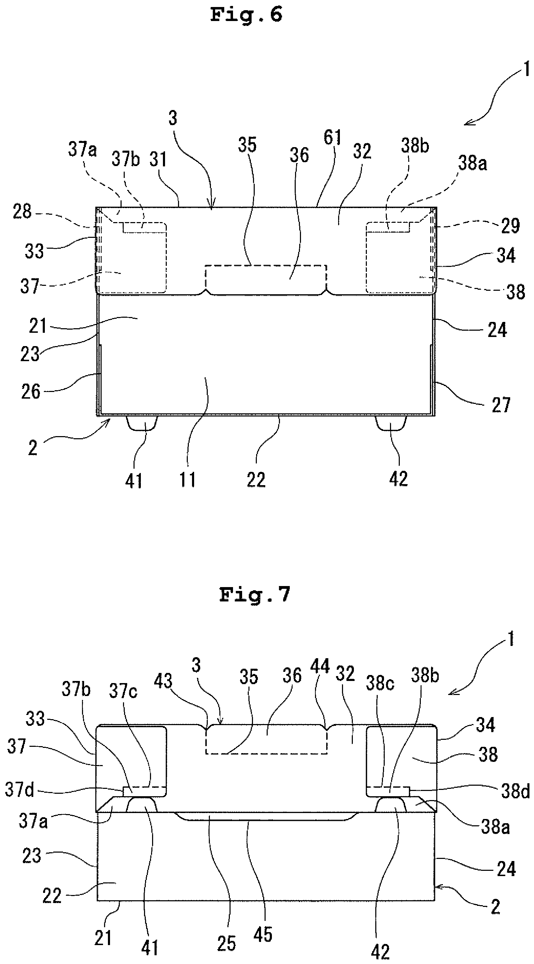

In a state where the lid 3 illustrated in FIG. 6 is opened to stand upright from the body 2 (the lid 3 pivots by 90 degrees), most of the storage 11 is opened, and the stored object stored on the front side can be taken out. In a state where the lid 3 illustrated in FIG. 8 is fully opened (the lid 3 pivots by 180 degrees), the storage 11 is entirely exposed, and all the stored objects can be taken out.

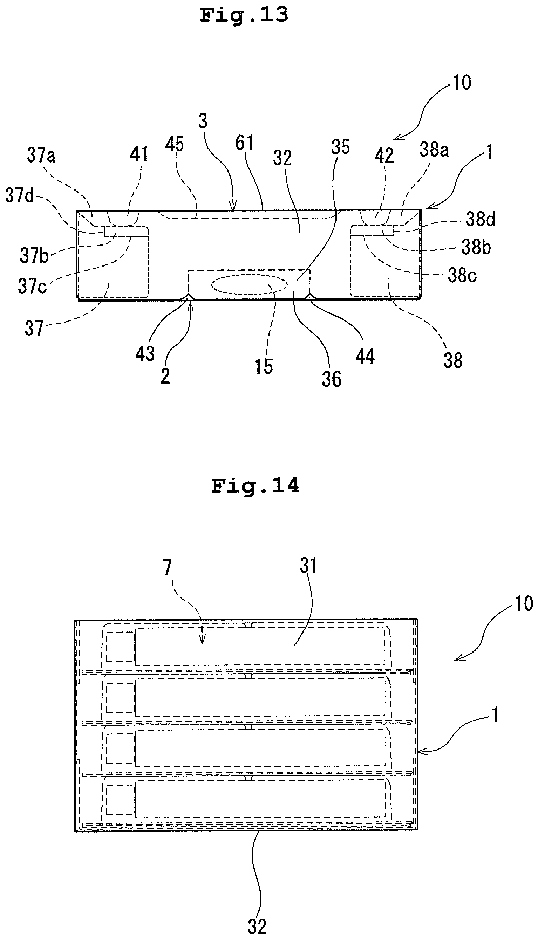

Next, a packaged medical instrument 10 of the present invention illustrated in FIGS. 13 to 16 will be described.

The packaged medical instrument 10 of the present invention includes: the packaging box 1 described above, and a medical instrument 7 stored in the packaging box 1, and has an opening of the body 2 of the packaging box 1 blocked by the lid 3, and the lid 3 is in a state of being bonded to a surface portion of the front surface part 22 of the body 2 overlapping the pressing portion for opening 36 on a rear surface of the pressing portion for opening 36.

As the packaging box 1, the above-described packaging box is used.

Examples of the medical instrument 7 may include a drug container storing a medicine such as drug, a prefilled syringe, or an injection needle. In particular, in this embodiment, as illustrated in FIG. 14, a plurality of, specifically, four individually packaged prefilled syringes 7 are stored in the packaging box 1. The individually packaged prefilled syringes 7 each include a prefilled syringe and a package that houses the prefilled syringe and is sealed.

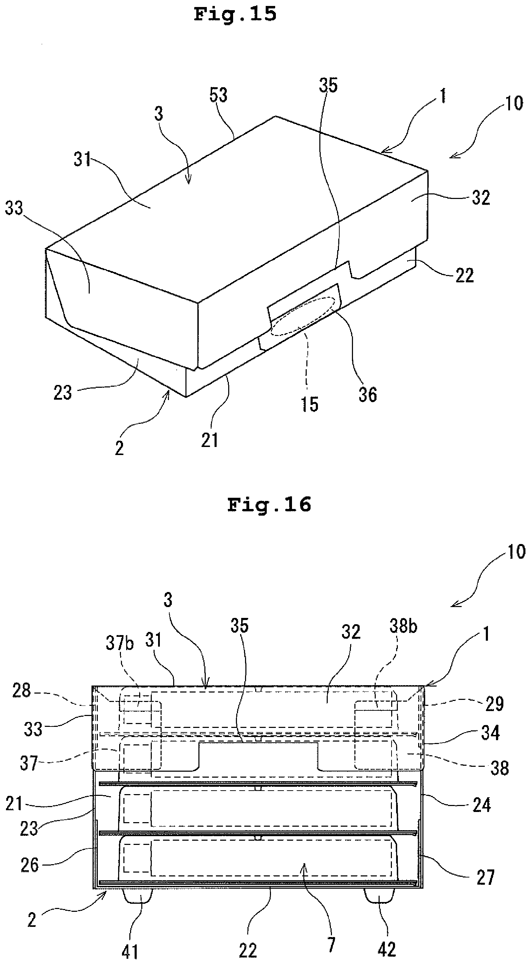

In the packaged medical instrument 10 of this embodiment, as illustrated in FIG. 13, the opening of the body 2 of the packaging box 1 is closed by the lid 3, and is, on the rear surface of the pressing portion for opening 36 of the lid 3, bonded with the surface portion of the front surface part 22 of the body 2 overlapping the pressing portion for opening 36 at a bonding part 15. Then, as illustrated in FIG. 15, by pressing the pressing portion for opening 36, the lid front surface part 32 of the lid 3 is broken at the breaking slit 35, the pressing portion for opening 36 is separated from the lid 3, and a fixed state between the lid 3 and the body 2 is released. The pressing portion for opening 36 separated from the lid 3 maintains a state of being fixed to the front surface part 22 of the body 2. When the lid 3 is pushed up (the opening operation is performed), the lid front surface part 32 of the lid 3 moves upward as illustrated in FIG. 15. By further pushing up the lid 3 (the opening operation is continued), as illustrated in FIG. 16, the lid 3 is in a standing state, and the stored object (medical instrument 7) can be taken out.

When the stored object (medical instrument 7) remains in the packaging box 1, an operation of closing the lid (lid closing operation) is performed. The lid closing operation is performed by pushing down the lid 3. By performing the lid closing operation, the lid 3 moves downward, and the bendable portions 37b and 38b pass through the protruding pieces 41 and 42. Then, by ending the downward pressing (pushing up operation) of the lid 3, the state illustrated in FIG. 9 is obtained, and the protruding pieces 41 and 42 of the body 2 enter the non-joint portion 39 of the lid 3. The upper ends of the bendable portions 37b and 38b abut on the lower surfaces of the protruding pieces 41 and 42, and the lid 3 is locked to the body 2 (lid locked state). Although there is a possibility that the state illustrated in FIG. 4 is obtained by ending the downward pressing (pushing up) of the lid 3, the lid 3 after being opened is normally in the lid locked state as illustrated in FIG. 9 since the lid 3 is slightly biased upward.

### INDUSTRIAL APPLICABILITY

The packaging box of the present invention is as follows.
(1) A packaging box including: a body having a storage whose upper side is opened; and a lid connected to the body and capable of opening and closing the opening, the packaging box capable of storing a plurality of stored objects, in which
   the body includes a bottom surface part, a front surface part rising from the bottom surface part, two side surface parts opposed to each other rising from the bottom surface part, and a back surface part rising from the bottom surface part and opposed to the front surface part,
   the lid includes a top surface part, a lid front surface part extending downward from the top surface part, two lid side surface parts opposed to each other extending downward from the top surface part, and two lid front side joints extending from tips of the two lid side surface parts, and the two lid front side joints are connected to inner surfaces of both side portions of the lid front surface part,
   when the lid is closed, the lid front surface part is positioned on a surface side of the front surface part of the body,
   the body further includes a protruding piece positioned on one side portion of the front surface part and extending from an upper edge of the front surface part,
   the lid front side joint includes a cutout portion provided at an upper end portion and extending from a base end side toward a distal end side of the lid front side joint by a predetermined length, and a bendable portion provided below the cutout portion, and a portion between the bendable portion and the lid front surface part is a non-joint portion into which a distal end of the protruding piece of the body is able to enter, and
   in the packaging box, when the lid is closed or when operating to open the lid, the protruding piece of the body enters the non-joint portion, and the protruding piece and the bendable portion abut on each other to bring the lid into a lid locked state, and when operating to open the lid after entering of the protruding piece, the bendable portion of the lid front side joint is bent by abutment with the protruding piece, pushes up the protruding piece at a bendable portion, and is able to release a contact state between the lid and the protruding piece, and further, when operating to close the lid, the lid front side joint comes into contact with the protruding piece, pushes down the protruding piece, and the protruding piece enters the non-joint portion of the lid, and the protruding piece and the bendable portion are engaged with each other, and a lid closed state is maintained.

   In this packaging box, the front surface part of the body includes a protruding piece positioned at one side portion of the front surface part and extending from the upper edge of the front surface part, the lid front side joint includes a cutout portion provided at the upper end portion and extending from the base end side toward the distal end side of the lid front side joint by a predetermined length, and a bendable portion provided below the cutout portion, and a portion between the bendable portion and the lid front surface part is a non-joint portion into which the distal end of the protruding piece of the body is able to enter.
   Therefore, at the time of an operation of closing the lid, the lid front side joint comes into contact with the protruding piece to push down the protruding piece, and the protruding piece enters the non-joint portion of the lid, and the protruding piece and the bendable portion are engaged with each other to maintain the lid closed state.
   The above aspect may be as follows.
(2) The packaging box according to the above-described (1), in which
   the front surface part of the body includes two protruding pieces positioned at one side portion and another end portion of the front surface part and extending from the upper edge of the front surface part, and
   each of the lid front side joints includes a cutout portion provided at the upper end portion and extending from the base end side toward the distal end side of the lid front side joint by a predetermined length, and a bendable portion provided below the cutout portion, and a portion between the bendable portion and the lid front surface part is a non-joint portion into which the distal end of the protruding piece of the body is able to enter.

   The packaging box of the present invention is as follows.
(3) A packaging box including: a body having a storage whose upper side is opened; and a lid connected to the body and capable of opening and closing the opening, the packaging box capable of storing a plurality of stored objects, in which
   the body includes a bottom surface part, a front surface part rising from the bottom surface part, two side surface parts opposed to each other rising from the bottom surface part, and a back surface part rising from the bottom surface part and opposed to the front surface part,
   the lid includes a top surface part, a lid front surface part extending downward from the top surface part, two lid side surface parts extending downward from the top surface part and opposed to each other, and two lid front side joints extending from both ends of the lid front surface part, and the two lid front side joints are joined to inner surfaces of distal ends of the two lid side surface parts,
   when the lid is closed, the lid front surface part is positioned on a surface side of the front surface part of the body,
   the body further includes a protruding piece positioned on a distal end of the one side portion and extending from an upper edge of the side surface part,
   the lid front side joint includes a cutout portion provided at an upper end portion and extending from a base end side toward a distal end side of the lid front side joint by a predetermined length, and a bendable portion provided below the cutout portion, and a portion between the bendable portion and the lid side surface part is a non-joint portion into which a distal end of the protruding piece of the body is able to enter, and
   in the packaging box, when the lid is closed or when operating to open the lid, the protruding piece of the body enters the non-joint portion, the protruding piece and the bendable portion abut on each other to bring the lid into a lid locked state, and when operating to open the lid after entering of the protruding piece, the bendable portion of the lid front side joint is bent by abutment with the protruding piece, pushes up the protruding piece at a bendable portion, and is able to release a contact state between the lid and the protruding piece, and further, when operating to close the lid, the lid front side joint comes into contact with the protruding piece, pushes down the protruding piece, and the protruding piece enters the non-joint portion of the lid, and the protruding piece and the bendable portion are engaged with each other, and a lid closed state is maintained.

   In this packaging box, the body includes a protruding piece positioned at a distal end of one side surface part and extending from the upper edge of the side surface part, the lid front side joint includes a cutout portion provided at the upper end portion and extending from the base end side toward the distal end side of the lid front side joint by a predetermined length, and a bendable portion provided below the cutout portion, and a portion between the bendable portion and the lid front surface part is a non-joint portion into which the distal end of the protruding piece of the body is able to enter.
   Therefore, at the time of an operation of closing the lid, the lid front side joint comes into contact with the protruding piece to push down the protruding piece, and the protruding piece enters the non-joint portion of the lid, and the protruding piece and the bendable portion are engaged with each other to maintain the lid closed state.
   The above aspect may be as follows.
(4) The packaging box according to the above-described (3), in which the body includes a protruding piece positioned at a distal end of each of the side surface part and extending from an upper edge of the side surface part, and
   each of the lid front side joints includes a cutout portion provided at an upper end portion and extending from a base end side toward a distal end side of the lid front side joint by a predetermined length, and a bendable portion provided below the cutout portion, and a portion between the bendable portion and the lid front surface part is a non-joint portion into which a distal end of the protruding piece of the body is able to enter.
(5) The packaging box according to any one of the above-described (1) to (4), in which the bendable portion includes a linear easily deformable portion formed substantially parallel to an upper edge of the cutout portion, and a longitudinal linear easily deformable portion extending from a base end side portion of the linear easily deformable portion toward the upper edge.
(6) The packaging box according to any one of the above-described (1) to (5), in which the body and the lid are connected to each other via a hinge line.
(7) The packaging box according to any one of the above-described (1) to (6), in which the body includes two back side joints extending from the back surface part or the side surface part and joining the back surface part and the side surface part.
(8) The packaging box according to any one of the above-described (1) to (7), in which the body and the lid include a hinge line at a boundary portion between the back surface part of the body and the top surface part of the lid, and are connected to each other via the hinge line.
(9) The packaging box according to any one of the above-described (1) to (8), in which the body includes two front side joints extending from the front surface part or the side surface part and joining the front surface part and the side surface part.
(10) The packaging box according to any one of the above-described (1) to (9), in which the lid front surface part includes a breaking slit having a distal and a terminal ends positioned at a lower end of the lid front surface part, and a pressing portion for opening defined by the breaking slit and a lower end of the lid front surface part, and the lid is bonded in a closed state to a rear surface of the pressing portion for opening at a surface portion of the front surface part of the body overlapping the pressing portion for opening.
   The medical instrument of the present invention is as follows.
(11) A packaged medical instrument including: the packaging box according to the above-described (10); and a plurality of medical instruments housed in the packaging box, in which the opening of the body of the packaging box is blocked by the lid, and the lid is in a state of being bonded to a surface portion of the front surface part of the body overlapping the pressing portion for opening on a rear surface of the pressing portion for opening.
   After the packaging box is opened, the medical instrument is taken out, and then the lid is closed, and the lid front side joint comes into contact with the protruding piece and pushes down the protruding piece, and the protruding piece enters the non-joint portion of the lid, the protruding piece and the bendable portion are engaged with each other, and the lid closed state is maintained, and it is possible to reliably bring the lid into the lid closed state and reliably maintain the lid closed state.
   The above aspect may be as follows.
(12) The packaged medical instrument according to the above-described (11), in which the medical instrument is a prefilled syringe or a drug storage container.

## Claims

1. A packaging box comprising: a body having a storage whose upper side is opened; and a lid connected to the body and capable of opening and closing the opening, the packaging box capable of storing a plurality of stored objects, wherein
the body includes a bottom surface part, a front surface part rising from the bottom surface part, two side surface parts opposed to each other rising from the bottom surface part, and a back surface part rising from the bottom surface part and opposed to the front surface part,
the lid includes a top surface part, a lid front surface part extending downward from the top surface part, two lid side surface parts opposed to each other extending downward from the top surface part, and two lid front side joints extending from tips of the two lid side surface parts, and the two lid front side joints are connected to inner surfaces of both side portions of the lid front surface part,
when the lid is closed, the lid front surface part is positioned on a surface side of the front surface part of the body,
the body further includes a protruding piece positioned on one side portion of the front surface part and extending from an upper edge of the front surface part,
the lid front side joint includes a cutout portion provided at an upper end portion and extending from a base end side toward a distal end side of the lid front side joint by a predetermined length, and a bendable portion provided below the cutout portion, and a portion between the bendable portion and the lid front surface part is a non-joint portion into which a distal end of the protruding piece of the body is able to enter, and
in the packaging box, when the lid is closed or when operating to open the lid, the protruding piece of the body enters the non-joint portion, and the protruding piece and the bendable portion abut on each other to bring the lid into a lid locked state, and when operating to open the lid after entering of the protruding piece, the bendable portion of the lid front side joint is bent by abutment with the protruding piece, pushes up the protruding piece at a bendable portion, and is capable of releasing a contact state between the lid and the protruding piece, and further, when operating to close the lid, the lid front side joint comes into contact with the protruding piece, pushes down the protruding piece, and the protruding piece enters the non-joint portion of the lid, and the protruding piece and the bendable portion are engaged with each other, and a lid closed state is maintained.

2. The packaging box according to claim 1, wherein
the front surface part of the body includes two protruding pieces positioned at one side portion and another end portion of the front surface part, and extending from the upper edge of the front surface part, and
each of the lid front side joints includes a cutout portion provided at an upper end portion and extending from a base end side toward a distal end side of the lid front side joint by a predetermined length, and a bendable portion provided below the cutout portion, and a portion between the bendable portion and the lid front surface part is a non-joint portion into which a distal end of the protruding piece of the body is able to enter.

3. A packaging box comprising: a body having a storage whose upper side is opened; and a lid connected to the body and capable of opening and closing the opening, the packaging box capable of storing a plurality of stored objects, wherein
the body includes a bottom surface part, a front surface part rising from the bottom surface part, two side surface parts opposed to each other rising from the bottom surface part, and a back surface part rising from the bottom surface part and opposed to the front surface part,
the lid includes a top surface part, a lid front surface part extending downward from the top surface part, two lid side surface parts extending downward from the top surface part and opposed to each other, and two lid front side joints extending from both ends of the lid front surface part, and the two lid front side joints are joined to inner surfaces of distal ends of the two lid side surface parts,
when the lid is closed, the lid front surface part is positioned on a surface side of the front surface part of the body,
the body further includes a protruding piece positioned on a distal end of the one side portion and extending from an upper edge of the side surface part,
the lid front side joint includes a cutout portion provided at an upper end portion and extending from a base end side toward a distal end side of the lid front side joint by a predetermined length, and a bendable portion provided below the cutout portion, and a portion between the bendable portion and the lid side surface part is a non-joint portion into which a distal end of the protruding piece of the body is able to enter, and
in the packaging box, when the lid is closed or when operating to open the lid, the protruding piece of the body enters the non-joint portion, the protruding piece and the bendable portion abut on each other to bring the lid into a lid locked state, and when operating to open the lid after entering of the protruding piece, the bendable portion of the lid front side joint is bent by abutment with the protruding piece, pushes up the protruding piece at a bendable portion, and is able to release a contact state between the lid and the protruding piece, and further, when operating to close the lid, the lid front side joint comes into contact with the protruding piece, pushes down the protruding piece, and the protruding piece enters the non-joint portion of the lid, and the protruding piece and the bendable portion are engaged with each other, and a lid closed state is maintained.

4. The packaging box according to claim 3, wherein
the body includes a protruding piece positioned at a distal end of each of the side surface part and extending from an upper edge of the side surface part, and
each of the lid front side joints includes a cutout portion provided at an upper end portion and extending from a base end side toward a distal end side of the lid front side joint by a predetermined length, and a bendable portion provided below the cutout portion, and a portion between the bendable portion and the lid front surface part is a non-joint portion into which a distal end of the protruding piece of the body is able to enter.

5. The packaging box according to any one of claims 1 to 4, wherein the bendable portion includes a linear easily deformable portion formed substantially parallel to an upper edge of the cutout portion, and a longitudinal linear easily deformable portion extending from a base end side portion of the linear easily deformable portion toward the upper edge.

6. The packaging box according to any one of claims 1 to 5, wherein the body and the lid are connected to each other via a hinge line.

7. The packaging box according to any one of claims 1 to 6, wherein the body includes two back side joints extending from the back surface part or the side surface part and joining the back surface part and the side surface part.

8. The packaging box according to any one of claims 1 to 7, wherein the body and the lid include a hinge line at a boundary portion between the back surface part of the body and the top surface part of the lid, and are connected to each other via the hinge line.

9. The packaging box according to any one of claims 1 to 8, wherein the body includes two front side joints extending from the front surface part or the side surface part and joining the front surface part and the side surface part.

10. The packaging box according to any one of claims 1 to 9, wherein the lid front surface part includes a breaking slit having a distal and a terminal ends positioned at a lower end of the lid front surface part, and a pressing portion for opening defined by the breaking slit and a lower end of the lid front surface part, and the lid is bonded in a closed state to a rear surface of the pressing portion for opening at a surface portion of the front surface part of the body overlapping the pressing portion for opening.

11. A packaged medical instrument comprising: the packaging box according to claim 10; and a plurality of medical instruments housed in the packaging box, wherein the opening of the body of the packaging box is blocked by the lid, and the lid is in a state of being bonded to a surface portion of the front surface part of the body overlapping the pressing portion for opening on a rear surface of the pressing portion for opening.

12. The packaged medical instrument according to claim 11, wherein the medical instrument is a prefilled syringe or a drug storage container.
